# EUROPEAN PATENT APPLICATION

(11) **EP 4 148 652 A1**
(43) Date of publication of application: **15.03.2023**
(21) Application number: 22182572.2
(22) Date of filing: 01.07.2022
(51) Int. Cl.: G06Q 50/02, A01K 11/00, A01K 29/00, A22C 17/00

(54) **METHOD AND SYSTEM FOR AUTOMATIC AND ENHANCED TRACEABILITY OF BEEF PRODUCTS FROM THE ORIGIN TO THE END CONSUMER**

(30) Priority: 02.07.2021 ES 202130621
(71) Applicant: Digitanimal, S.L., 28830 San Fernando de Henares, Madrid (ES)
(72) Inventor: Callejero Andrés, Carlos, 28850 Madrid (ES); Gómez Maqueda, Ignacio, 28051 Madrid (ES); Blanco Carrera, Rubén, 28028 Madrid (ES)
(74) Representative: Balder IP Law, S.L.

(57) **Abstract**

Method and system for generative animal traceability. The system comprises: monitoring devices (1) placed on the animals (100), communication devices (2a,2b) and computing devices (3). A first processor (12) of the monitoring device stores and transmits sensor measurements together with an animal identification; each communication device (2) determines its location through satellite and/or wireless communications, receives data from the monitoring devices (1), associates a specific location to the animal that carries the monitoring device and stores and transmits the data with its associated location; the computing devices (3) receive and process the data of the monitoring devices for generating animal traceability events, and transmit these events to one or more servers for its further query by the users.

## Description

### TECHNICAL FIELD

The present invention belongs to the technical field of electronic systems. More precisely, this invention belongs to the field of electronic systems for the full traceability of the animals from which a meat product has been obtained, from its birth to its distribution, potentially including information about management practices as a certificate seal of the livestock farm. This invention refers to a method and system that allows to share the traceability data of a particular animal from which a specific meat product is being consumed, e.g., end consumer, facilitating the transmission of the full traceability information from the birth of the animal to the distribution of the meat product.

### BACKGROUND

European citizens are increasingly demanding more information on animal welfare, traceability and the environmental impact of meat consumption. Moreover, the effects of COVID19 will accelerate the digitalization processes promoting directs sales from producers to consumers, given that this situation has already triggered a shift in consumer trends, some studies suggest that online sales have increased more than 15%.

The boost given by the European Commission to the "Farm to Fork Strategy" is focused on improving the sustainability of agrifood systems and guarantee the provision of healthy and affordable food products for all European citizens. Besides that, there exists an important demand of more sustainable products in the European market and the consumers aware of these issues want to have the assurance that food products comply with sustainability requirements.

Three highly differentiated production stages are identified in the meat production chain, each one having its own management challenges:
- Breeding stage associated breeding farms: this stage includes the birth of the animal and the first months of its life. In the case of cattle, calves are fed with breast milk and grass during the first months of their lives.
- Fattening stage associated with feedlots: this is an intensive livestock production focused on fattening livestock in indoor stables.
- Slaughtering and processing stage: when the animal has reached the desired weight it is sent to the slaughterhouse to later proceed with cutting, ageing and packaging the final product. Finally, the distribution to the consumer is achieved through different intermediary chains.

In all the EU countries, meat traceability is regulated by European legislations such as the Regulation (EU) No 1308/2013 of the European Parliament and of the Council of 17 December 2013 establishing a common organisation of the markets in agricultural products and repealing Council Regulations (EEC) No 922/72, (EEC) No 234/79, (EC) No 1037/2001 and (EC) No 1234/2007, Commission Delegated Regulation (EU) 2017/1182 of 20 April 2017 supplementing Regulation (EU) No 1308/2013 of the European Parliament and of the Council as regards the Union scales for the classification of beef, pig and sheep carcasses and as regards the reporting of market prices of certain categories of carcasses and live animals and Regulation (EC) No 1760/2000 of the European Parliament and of the Council of 17 July 2000 establishing a system for the identification and registration of bovine animals and regarding the labelling of beef and beef products and repealing Council Regulation (EC) No 820/97, among others.

The main pillars for meat traceability established in these regulations are the following:
1. The identification and registration system of the animals is the first link for meat traceability, from birth to slaughtering. For the consumers, it ensures the innocuousness of meat products and the acknowledgement of their origin, but it is also essential for livestock farmers since it facilitates transactions, tracking the production, managing the herd's health and protection against thefts. This identification and registration is done with several elements:
   ∘ Ear tags (plastic tags placed on the animal's ear): in cattle, usually two ear tags, one on each ear are used, with the same unique code that allows to individually identify each animal and farm where it has been born.
   ∘ Computerized databased system used for all live livestock, from its birth to its slaughtering. This system integrates three modules: the General Register of Livestock Farms, the Individual Animal Identification Register and the Movement Register. Data originates from the manual registration of livestock farmers, that manage animal additions and removals, issue identification documents, declare their animals' census, etc., and from the regional authorities responsible for inspections, controls, document issuing, etc.
   ∘ Cattle Identification Document: cattle passport that registers all the information from its birth. This document contains the following information: date of birth, gender, breed, mother's id (as reflected on her ear tag), place of birth (farm id), day of entry to the farm, slaughtering date and movements (date, farm of origin and farm of destination).
   ∘ Farm Ledger: register of all entries and exits on the farm, remarks of inspections, incidences (such as ear tag changes), etc. It used to be done by hand with the official farm registers that are still used by livestock farmers, however, they are increasingly adopting the use of digitalized registers such as excel files. This register must be accessible to the competent authority for a minimum period of three years.
2. The compulsory label of the origin of the meat products, which goes from slaughtering to when the meat product reaches the end consumer. Once the animal arrives to the slaughterhouse, it is mandatory and under strict control the verification of the animal's identity through the match of the ear tag code and the Bovine Identification Document (BID). If everything is correct, once the animal is slaughtered, a reference number is assigned to the carcass, together with the sanitary authorization number of the slaughterhouse. After the slaughtering process, the carcass is sent to the cutting plant where a sanitary authorization number is also issued. Following the current regulations, the labels of meat products must include:
   ∘ Reference number: this is the number assigned to the slaughtered animals, which links the meat product with the animal or animals from which the meat product has been obtained.
   ∘ Country of birth: the one indicated on the ear tags.
   ∘ Country where the animal has been raised or fattened: the one indicated on the Cattle Identification Document and Farm Register.
   ∘ Country of slaughtering and sanitary authorization number of the slaughterhouse: the one indicated on the documents issued on the slaughterhouse.
   ∘ Country of cutting and sanitary authorization number of the cutting plant: the one indicated on the documents issued on the cutting plant.

As mentioned beforehand, there are market trends that are not defined in the current regulations and is not possible to verify its compliance (pasture raised beef, local production, km0 meat, etc.). It is also not possible to transmit information on what is happening on the farm, the conditions of transportation of live animals and the packaging of meat products, what is especially difficult in the case of burgers.

Over the last years, intelligent tools have been developed in order to help livestock farmers to optimise their operations, developing monitoring devices for tracking the health and behaviour of the animals. However, these intelligent systems are lacking the integration of standard data, require a costly implementation without a guaranteed return on investment and need improved tools that are easier to use for livestock farmers that are not usually trained on these technologies.

The current stage of development of Internet of Things devices together with distributed computing and the development of algorithms using machine learning and big data potentially allows the gathering, generation, analysis and management of data along the whole meat production chain. The use of these tools, especially at large scale, could help the livestock sector to become more efficient and achieve the animal welfare and environmental sustainability goals. Furthermore, the use of digital ledgers (e.g., blockchain) could increase transparency when reaching the end consumer.

Some of the developments in the state of the art of this subject are:
- US2020184416A y US2019303846A (where systems for registering information on the different stages through which a biological object passes by either by passing through different locations or thanks to a device included in the specific object are presented). These systems do not allow to perform the traceability of beef products as proposed as they do not allow the inclusion of information coming from elements external to the object; they do not allow either the communication between different agents in the chain, specifically looking back (from the end consumer to the producer). They also do not aim to show information about good management practices and animal welfare to the other agents in the chain.
- BR102018069615A introduces a device for tracing and monitoring dairy production. Meat and dairy production systems are completely different both in the origin (farm) and in the processing step; the described device does not have the capacity to achieve the full traceability when it is a meat product.
- There are other developments such as WO20010159A1 where a specific method for storing information is described. Lastly, documents such as WO19174952A1 y US2009224873A refer to specific methods for encoding or exchanging information for tracking the products.

### DESCRIPTION OF THE INVENTION

The present invention aims to solve some problems that present state of the art solutions as those above-mentioned.

Thus, the present invention consists of a method and system for automatic and enhanced traceability of beef products during a period of time or between two locations. The first location or the start of the period of time preferably comprises the origin in intensive or extensive livestock farms and the second location or the end of the period of time preferably includes the distribution and more preferably, when the end consumer already owns the meat product therefore, it is possible to achieve the traceability from the birth of the animal to its slaughtering and even after.

A first aspect of the invention refers to a system for generating traceability of an animal, comprising: one or more monitoring devices, each one adapted for being secured to the animal; one or more communication devices; and one or more computing devices. Each monitoring device comprises: a first sensor for measuring one or more physical quantities of the animal and/or of an environment in which it is located when the monitoring device is secured to the animal; a first wireless communication module for data transmission and reception; a first processor; and a first storage unit; the first processor being configured for storing measurements of the first sensor and at least one identifier of the animal in the first storage unit, and for transmitting at least measurements of the first sensor and the identifier of the animal to the communication device intermittently and/or after receiving an interrogation from the communication device. Each communication device comprises: a positioning device configured for determining its position by means of satellites and/or wireless communications; a second wireless communication module for data transmission and reception; a second processor; and a second storage unit; the second processor being configured for receiving transmissions from the monitoring device when it is in a coverage area of the monitoring device, for associating a position determined by the positioning device with the animal of the monitoring device every time transmissions are received, for storing transmissions with an associated position in the second storage unit, and for transmitting at least transmissions with an associated position to the one or more computing devices or to a data network to which the one or more computing devices are communicated. The one or more computing devices comprise: a communication module for data transmission and reception; and one or more processors; the one or more processors being configured for processing transmissions of the monitoring device such that, based on at least the measurements contained in the transmissions and the associated positions, it generates traceability events associated with the animal by means of the identifier of the animal, and for transmitting the traceability events associated with the animal to one or more servers.

Each monitoring device monitors one or more physical magnitudes of the animal and/or the environment when the respective animal is carrying the monitoring device with the aim of knowing what is the status of the animal at different points in time throughout the monitoring carried out for its traceability.

With the goal of reducing its power consumption and the complexity of the monitoring devices therefore minimizing the maintenance tasks and their manufacturing costs. For knowing the location of the animal at different points in time throughout the monitoring carried out for its traceability, the monitoring devices preferably do not include a positioning device and takes advantage of the positioning device of the communication device with which it communicates. The location determined by the communication device can be attributed to the location of the monitoring device, as the transmission between the monitoring device and the communication device can only be done when the two devices are close to each other. This is due to the fact that the first wireless communication module needs to communicate with the second communication module, that is only possible at short distances.

For reducing the power consumption and the complexity of the monitoring device, the monitoring devices do not have a wireless communication module for communicating with one or more computing devices that carry out the data processing needs in order to generate the animal traceability. In this regard, each monitoring device sends the data corresponding to each monitoring period to one or more communicating devices and the communication devices that receive the data, process it and send it to one or more computing devices with other data, such as the location.

In some embodiments, each monitoring device also comprises a positioning device set up for determining its location through satellite and/or wireless networks.

Occasionally it can be useful to know the location of each monitoring device even when they are not close to a communication device, despite the monitoring device being more complex and increasing its power consumption.

Nevertheless, in other embodiments, each monitoring device determines its location using one or more inertial sensors, or through triangulation based on the wireless communications received or transmitted with the first wireless communication module.

In some embodiments, the animal is a first animal; at least one of the one or more communication devices is adapted for being secured to a second animal and further comprises a second sensor for measuring one or more physical quantities of the second animal and/or of an environment in which it is located when said at least one communication device is secured to the second animal; the second processor of said at least one communication device is additionally configured for storing measurements of the second sensor and at least one identifier of the second animal in the second storage unit, and for transmitting at least the measurements of the second sensor and the identifier of the second animal with an associated position to the one or more computing devices or to a data network to which the one or more computing devices are communicated; the one or more processors are configured for processing the measurements of the second sensor and the associated positions and generating traceability events associated with the second animal by means of the identifier of the second animal, and for transmitting the traceability events associated with the second animal in the one or more servers.

Therefore, the communication devices can be at the same time monitoring devices with communication capacities additional to the ones provided by monitoring devices.

Preferably, one or more monitoring devices are assigned to one or more calves and one or more communicating devices are assigned to animals that are related to one or more calves, for instance, the calf's mother. Calves tend to be close to their mothers and in fact, the possibility that only the animals that have monitoring devices can be tracked when they are close to some communication devices is indicative that the communication devices (and, therefore, the calves, following this example) have stayed close to the communication devices (and, therefore, their mothers, following this example); this is specially clear in the breeding stage, but it can also be observed in other stages of the animals lives. The reduction of manufacturing and maintenance costs is convenient given that there are more calves than mothers in a farm, and a reducing complexity in the monitoring devices results in a lighter device used by the calves.

In some embodiments, at least one of the one or more communication devices further comprises one or more second sensors for measuring one or more physical quantities of the animal and/or of an environment in which it is located when the animal actuates or activates the one or more second sensors and/or for measuring one or more physical quantities of an installation or vehicle; the second processor of said at least one communication device is additionally configured for interrogating each of the one or more monitoring devices at least when it detects that it is in a coverage area of each respective monitoring device, and is configured for transmitting, together with transmissions with an associated position, measurements of the one or more second sensors; and the one or more processors are configured for generating the traceability events also based on the measurements of the one or more second sensors.

When one or more communication devices are assigned to an area or object of a facility, for instance, a farm or a slaughterhouse, from such facility the communication is carried out with one or more computing devices. The passing or presence of animals in such facilities is frequent or continuous. In addition, the communication devices can have a fixed power supply, including the electric network rather than batteries.

Likewise, when one or more communication devices are deployed in one or more facilities, the second sensor or group of sensors included in the monitoring devices register additional physical or environmental magnitudes to the ones registered by the sensor or group of sensors of the first monitoring device. When the one or more communication devices are installed in one or more facilities, the one or more second sensors measure physical magnitudes of the animal and/or the environment, in addition to the ones that the first sensor of the monitoring device has measured, therefore, more data is available on the animal and its environment for its traceability, and/or the facility where it is located (e.g. temperature, humidity, gas concentration, animal weight, access to water and feed, etc.). These measurements are also sent to the one or more computing devices with the aim of generating traceability events based on such measurements.

It is possible to have one or more communication devices on the animals and one or more communication devices in the facilities for enhanced and more detailed animal traceability. That is, in some embodiments, at least one of the one or more communication devices are adapted for being placed on a second animal and comprises the second sensor for measuring one or more physical magnitudes of the second animal and/or of the environment when at least one communication device is placed on the second animal, and at least one other from the one or more communication devices comprises additional sensors for measuring one or more physical magnitudes and/or of the environment when the animal actuates or activates the one or more additional sensors and/or for measuring one or more physical magnitudes of a facility or vehicle.

In some embodiments, the system also comprises the facility or vehicle, comprising of any of these: a farm, a slaughterhouse, a cutting plant or a vehicle for the transportation of animals or meat products.

The animal's traceability can be done when the animals is alive and even when the animal has already been slaughtered, quartered and/or when being transported for sale.

In some embodiments, one or more servers are set up for serving the traceability events stored in it when an electronic query is received, included the electronic query o fan animals' identification.

A user can check the different traceability events of an animal when requested to one or more servers. In this query, the user must provide the animal's identification, that is, any digital code that identifies the particular animal.

In some embodiments, the one or more servers are configured for providing a digital ledger; and the one or more processors are configured for transmitting each traceability event associated with an animal to the one or more servers as a record in the digital ledger. In some of these embodiments. In some of these embodiments, the digital ledger is based on a blockchain like e.g. Blockchain.

With a digital ledger it is more difficult to alter traceability events if, for instance, a third party would try to modify or delete records that were already on the digital ledger. The alteration complexity increases when the digital ledger is based on Blockchain technology and its management is decentralized, as known in the state of the art.

In some embodiments, the one or more computing devices are one or more first computing devices; the system further comprises one or more second computing devices; the one or more second computing devices comprise: a communication module for data transmission and reception; one or more processors; and one or more storage units wherein a predetermined set of thresholds is stored; the one or more processors being configured for downloading the traceability events associated with an animal by means of the identifier of the animal, for processing the downloaded events such that they are compared with the predetermined set of thresholds, for generating a digital certification associated with the animal indicative of the comparison performed, and for transmitting the digital certification associated with the animal to the one or more servers and/or for storing the digital certification associated with the animal in the one or more storage units of the one or more second computing devices.

Digital certifications allow the identification of animals that have (or not) a traceability under a group of parameters defined by the group of predefined thresholds. In this way, the user can know from the digital certification what has been the evolution of the animal without the need to check the traceability events. This is especially advantageous for reducing the time and resources needed by the user in order to check the evolution of the animal because it is enough to perceive (e.g., visually) that the digital certification exists. For example, the product to be sold can include on or more seals that correspond to one or more digital certifications indicative of, for instance, what feed did the animal have, if the animal has met specific welfare standards, if the animal has stayed in the same place throughout its life, if the animal has been feed only with grass, if the meat has been produced locally, etc.

In some embodiments, the one or more servers is configured for serving digital certifications stored therein upon receiving an electronic request for digital certification consultation, the electronic request including an identification of the animal.

A user can check digitally an animal's certifications through the animal's identification. For instance, the user can introduce or scan a code corresponding to the animal's identification in a webpage or mobile app for carrying out the digital inquiry of the digital certification to the one or more servers.

In some embodiments, the wireless communication module of each monitoring device is a short-range wireless communication module adapted for data transmission and reception through a first communication protocol; the wireless communication module of each communication device is adapted for data transmission and reception through a first communication protocol and through a second communication protocol, data transmission and reception with the respective monitoring device is carried out through the first communication protocol and the data transmission and reception with the one or more computing devices is done through the second communication protocol.

Each monitoring device communicates with one or more communication devices through the first protocol, that is a short-range communication protocol (e.g., RFID, Bluetooth, ZigBee, WLAN, etc.) for reducing power consumption while each communication device also communicates with one or more computing devices through the second protocol that is preferably a long-range communication protocol and, optionally, is able to connect to the internet, e.g., UMTS, LTE, WLAN, LPWAN (Sigfox, NBIoT, LoRa), etc.

In some embodiments, the system also comprises one or more servers.

In some embodiments, the system also comprises one, several or all from: one or more electronic devices for data transmission for the generation of traceability events, one or more labels and/or codes adapted for being assigned to the animal, and one or more electronic devices for traceability management (that allows to transmit data, corresponding to an animal, to the one or more computing devices even when the animal is not carrying the monitoring device).

A second aspect of the invention refers to a method for generating traceability of an animal, comprising: placing each of one or more monitoring devices on an animal to be traced; measuring, with a first sensor of each monitoring device, one or more physical quantities of the animal and/or of an environment in which it is located when the monitoring device is placed on the respective animal; storing at least the measurements of the first sensor and an identifier of the animal in a first storage unit of the respective monitoring device; transmitting, from each monitoring device to one or more communication devices wirelessly by means of respective wireless communication modules for data transmission and reception, at least the measurement transmissions of the first sensor and the identifier of the respective animal intermittently and/or after receiving an interrogation from the communication device; determining, at least intermittently, the position of each communication device by means of a positioning device of the respective communication device, said determination being by means of satellites and/or wireless communications; storing, in a second storage unit of each communication device, the transmissions received from each monitoring device together with a determined position; transmitting, from each communication device to one or more computing devices or to a data network to which the one or more computing devices are communicated, at least the transmissions received together with a determined position; the one or more computing devices generating traceability events associated with the animal, by means of the identifier of the respective animal, based on the transmissions received from the respective monitoring device; and the one or more computing devices transmitting the traceability events associated with each animal to one or more servers.

This method allows for tracing one or more animals. Each of them has one or more physical magnitudes measured over time, as well as its location identified through one or more communication devices with which they communicate for transmitting, at least, the data related to the measurements carried out by its monitoring device.

Each communication device transmits the measurements and location together with the animal's identification to the one or more computing devices. The one or more computing device processes the collected data to generate traceability events in accordance with a set of predefined rules stored in a memory compartment of such computing devices or in one or more of the servers with which they communicate and where the traceability events are stored.

In some embodiments, each animal to be traced is a first animal to be traced; the method further comprising: placing each of the one or more communication devices on a second animal to be traced; measuring, with a second sensor of each communication device, one or more physical quantities of the second animal and/or of an environment in which it is located when the communication device is placed on the respective second animal; storing at least the measurements of the second sensor and an identifier of the second animal in the second storage unit of the respective communication device; transmitting, from each communication device to the one or more computing devices or to the data network to which the one or more computing devices are communicated, at least the measurements of the second sensor together with a determined position and the identifier of the respective second animal; the one or more computing devices generating traceability events associated with the second animal, by means of the identifier of the respective second animal, based on the transmissions received from the respective monitoring device; and the one or more computing devices transmitting the traceability events associated with each second animal to the one or more servers.

In some embodiments, each first animal is a calf and each second animal is a mother of a calf.

In some embodiments, the method further comprises: measuring, with one or more second sensors of one or more communication device, one or more physical quantities of the animal and/or of an environment in which it is located with a monitoring device placed when the animal actuates or activates the one or more second sensors and/or one or more physical quantities of an installation or vehicle; and interrogating each of the one or more monitoring devices with each of the one or more communication devices at least when the respective communication device detects that it is in a coverage area of the respective monitoring device; and in the step of transmitting at least the transmissions received together with a determined position, measurements of the one or more second sensors are also transmitted.

In some embodiments, the stage of measuring with one or more second sensors from each communication device comprises at least the measurement of the one or more physical magnitudes of the facility or vehicle; and the facility or vehicle comprises any of: a farm, a slaughterhouse, a cutting plant or a vehicle for the transportation of live animals or meat products.

In some embodiments, the method further comprising: transmitting, from an electronic device with a communication module (wired or wireless) to networks (e.g. a cellular network, Internet, etc.) for data transmission and reception, an electronic request for traceability event consultation including an identification of the animal; and the electronic device receiving from the one or more servers traceability events associated with the identification of the animal of the electronic request for consultation received.

In some embodiments, the one or more servers are set up to provide a digital ledger preferably based on Blockchain and the traceability events of each stage are transmitted as a record in the digital ledger.

In some embodiments, the one or more computing devices are one or more first computing devices; the method further comprising: one or more second computing devices of the one or more servers receiving the traceability events associated with an animal by means of the identifier of the animal; the one or more second computing devices processing the events received such that they are compared with a predetermined set of thresholds; the one or more second computing devices generating a digital certification associated with the animal indicative of the comparison performed; and the one or more second computing devices transmitting the digital certification associated with the animal to the one or more servers and/or storing the digital certification associated with the animal in one or more storage units of the one or more second computing devices.

In some embodiments, in the step of transmitting and/or storing the digital certification, the digital certification is at least transmitted to the one or more servers; and the method further comprising: transmitting, from an electronic device with a communication module (wired or wireless) to networks (e.g. a cellular network, Internet, etc.) for data transmission and reception, an electronic request for digital certification consultation including an identification of the animal; and the electronic device receiving from the one or more servers the digital certificate associated with the identification of the animal of the electronic request for consultation received.

In some embodiments, the stage of placing one of the one or more monitoring device is done within a week (that is, 168 hours), and preferably within 48 hours from the birth of the corresponding animal.

In some embodiments, the traceability events comprise one or more of: birth, movement of the animal between farms and premises, weaning of the animal, events relating to the health and welfare of the animal, entrance of the animal in a feedlot, movement of the animal in the feedlot, exit of the animal from the feedlot, transport of the live animal, slaughter, generation of meat product, and transport of the meat product.

In some embodiments, the wireless transmission of each monitoring device to the one or more communication devices is carried out when a distance between the respective monitoring device and the respective communication device is less than or equal to 500 meters (preferably less than or equal to 100 meters); and the transmission of each communication device to the one or more computing devices or to the data network is through a mobile communication network or the Internet.

Similar advantages as the ones described in relation with the first aspect of the invention are applicable to the second aspect of the invention.

### BRIEF DESCRIPTION OF THE FIGURES

With the aim of facilitating the comprehension of the features of this invention in accordance with examples of practical embodiments of the invention, a set of drawings is attached as an integral part of the description, in which, for illustrative and non-limiting purposes, the following has been depicted a set of drawings:
Figure 1 shows diagrammatically a system in accordance with some embodiments of the invention.
Figures 2 and 3 show diagrammatical monitoring and communicating devices, respectively, in accordance with some embodiments of the invention.

### DESCRIPTION OF EMBODIMENTS OF THE INVENTION

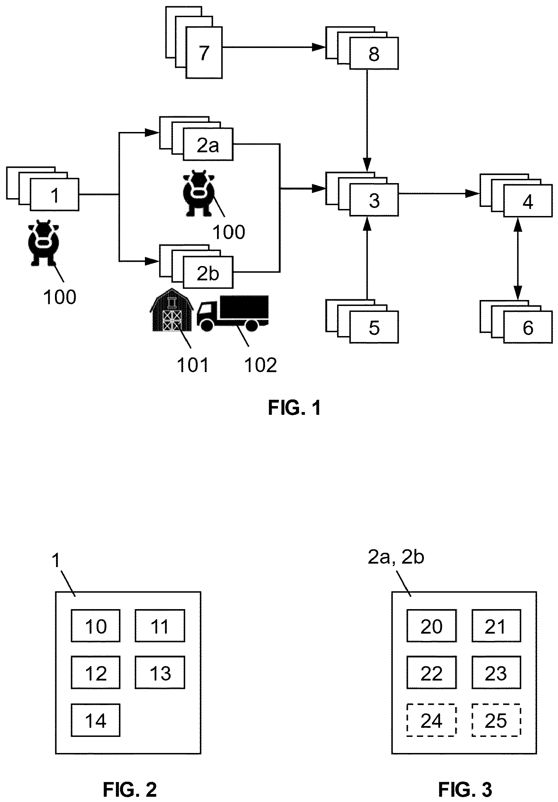

Figure 1 shows diagrammatically a system in accordance with some embodiments of the invention.

The system comprises one or more monitoring devices 1, one or more monitoring devices 2a, 2b, and one or more computing devices 3. In some embodiments, the system comprises one or more servers 4, and/or one or more electronic devices 5 for the transmission of information used for the generation of traceability events, and/or one or more electronic devices 6 for the transmission of electronic queries by the users (any agent in the chain interested in knowing the traceability of the animals) for receiving the traceability of one or more servers 4; these devices can include optical methods for code recognition and/or input means from the user with the goal of recognizing or introducing an identification of an animal from which the traceability events or digital certificates is to be known. Additionally, or alternatively, the systems can include one or more labels and/or codes 7, and one or more electronic devices 8 for traceability management that can include optical means for code recognition and/or input means by the user.

Each monitoring device 1 can be placed on an animal 100a as well as on a calf for keeping it secured. For this purpose, each monitoring device 1 comprises means to attach it such as a collar, an earring, a textile element adapted for holding it in any part of animal's 100a body, etc. In the same way, one or more of the communication devices 2a can be placed on a 100 animal such as a calf, preferably a calf's mother, for keeping it attached with similar means of attachment. Additionally, or alternatively, one or more communication devices 2b are installed on an area or object of a facility 101 or vehicle 102, for this purpose, they may have means of attachment such as adhesive, screws, etc, or not have means of attachment, in which case it rests on the object or Surface or any of these include means of attachment of device 2b, such as a case.

The monitoring devices 1 include an identification of the animal that is wearing it. The animal identification can be introduced either manually by a person, for instance, by means of user input as buttons or clicks, by an electronic device that communicates with monitoring device 1 for registering the animal identification from the electronic device, or it can even be a predefined value in the monitoring device, that is, the animal identification can be registered at the time when the monitoring device is being manufactured. This animal identification can be stored either in the monitoring device 1 storage or in the device's hardware.

In the same way that the animal's identification is introduced manually, events can also be introduced manually, as an example, animal health and welfare events can be registered: if an animal is sick, if it has received any drugs, when has it recovered, etc. These same events are treated as measurements and the monitoring device 1 can send them wirelessly in packages to a communication device for their further dispatch to the computing devices and the generation of traceability events.

Monitoring device 1 measures one or more physical magnitude of animal 100 and/or its environment while it carries the monitoring device 1. Measurements can be done continuously or intermittently, the time between two subsequent measurements can be set up for promoting energy savings. Each monitoring device 1 can also send either continuously or intermittently packages wirelessly to a communication device 2a, 2b when it is closed to any of them or when it is requested by any of them. The proximity required between both devices depends on the wireless communication module and the communication protocol used, short-range protocols are preferably used to reduce power consumption.

Packages sent include, at least, the animal's identification and one or more measurements, which could have been processed before their dispatch with the aim of extracting relevant information and send only this information. As an example, if fifty pulse measurements have been done in animal 100 and all of them lie within a specific range determined as healthy and calmed, the package can include a label that indicated that the animal is healthy and calmed.

The communication device 2a, 2b that receives packages from a monitoring device 1, sends, continuously or intermittently, the data received together with one location to the one or more computing devices 3. For the location, the communication device 2a, 2b records its own location by means of a positioning device included therein. Positioning is preferably obtained in a temporal frame in which the reception of the package from the corresponding monitoring device is done, being the time frame. This is, the positioning of communication device 2a, 2b can also be obtained before receiving the package as long as both moments (obtention of the location and reception of the package) are within the same time frame. The packages can be sent to the one or more computing devices 2 immediately after receiving the packages of monitoring device 1 or later, for instance, each time that several packages are ready to be sent.

The one or more computing devices 3 process all the packages received from the one or more communication devices 2a, 2b and generates traceability events for each respective animal based on the content f one or more packages associated with such animal.

The traceability events generated can be influenced by data sources external to the system. For instance, one or more electronic devices 5 can send to the one or more computing devices 3 (or be the computing devices 3 the ones that request information to the one or more electronic devices 5) information such as: animal registers following the current legislation, data sources that collect information from agro-environmental variables and other data sources that are relevant for enhancing the traceability of meat products.

Traceability events are sent to the one or more servers 4 for its further request from a user, if desired, and proceeds to carry out electronic queries from an electronic device 6 with ability to communicate wirelessly or trough cable with the one or more servers 4.

As an example, traceability events can be any of these:
- Farm events:
   ∘ Breeding stage
      ▪ Calf's birth: date, weight, location (coordinates, farm, country, etc.), mother's and calf's identification number.
      ▪ Animal movements between farms or paddocks: date, location and animal's identification number.
      ▪ Weaning (process when the calf is detached from the mother): date, event, location (coordinates, farm, country, etc.), mother's and calf's identification number.
      ▪ Health events (vaccinations, medical treatments, etc.) both from the mother and the calf: date, event, location (coordinates, farm, country, etc.), mother's and calf's identification number.
      ▪ Animal welfare events (access to water and feed, etc.) both from the mother and the calf: date, event, location (coordinates, farm, country, etc.), mother's and calf's identification number.
   Fattening stage:
      ▪ Entrada de animal en el cebadero: peso, fecha, identificacion, etc.
      ▪ Movements of animal, as in the previous stage, adapted to the conditions of the feedlot.
      ▪ Health events, as in the previous stage, adapted to the conditions of the feedlot.
      ▪ Animal welfare events, as in the previous stage, adapted to the conditions of the feedlot.
      ▪ Feedlot exit: weight, date, identification, etc.
- Events external to the farm (meat product):
   ∘ Slaughterhouse
      ▪ Monitorization prior to slaughtering: environmental conditions, waiting time, stress level of the animal, etc.
      ▪ Generation of the meat product from the animal: animal-carcass ratio (unprocessed meat product).
   ∘ Cutting plants
      ▪ Products generated from the carcass
      ▪ Nutritional analysis of the meat products
   ∘ Transportation
      ▪ Transportation density: number of animals
      ▪ Conditions of the transportation environment: temperature, humidity, gases, etc.
      ▪ Duration and location of transportation
   Distribution
      ▪ Location of the meat products

The generated events include the timestamp in which they take place and are associated with the corresponding animal through its identification. Likewise, these events preferably also include the physical entities involved (farm, feedlot, transportation, slaughterhouse and cutting plant).

Hereafter, the functioning of the traceability system and method in accordance with some embodiments of the invention. It will be noted that in other embodiments not each and every one of the traceability stages are included.

At the time of birth, a monitoring device 1 is installed on the animal. This device will be kept throughout all its life until slaughtering and it will communicate with one or more communication devices 2a, 2b for generating intermittently, by means of one or more computing devices 3, traceability events that will be transmitted to one or more servers 5 for its storage. Thus, a first animal birth event is generated.

Throughout the breeding stage, extensive environment with very few infrastructures, monitoring devices 1 and communication devices 2^{a}, 2b send information that is processed for generating traceability events such as movements between paddocks, health issues, etc. Once the breeding process has ended, the animal is transported to the feedlot generating traceability events generating additional traceability events in this process.

Once in the feedlot, monitoring devices 1 and computing devices 2a and 2b generate information that is processed in search of feedlot events. If this happens, the corresponding traceability events will be generated and transmitted for its storage, allowing for later access by the agents in the chain.

When the animal is being transported to the slaughterhouse, the process can also be monitored, as well as the arrival to the waiting area of the slaughterhouse and the slaughtering process where the carcass is obtained, which is the part of the animal from which meat products are generated. On this stages, transportation and slaughtering events are generated. At the time of slaughtering, the monitoring device 1 can be removed and instead, electronic tags (RFID) and/or codes 7 (such as barcodes or QR codes) can be installed. In the case of installing such labels or codes 7, they must be linked to the animal's identifiers in order to facilitate access to the previously generated information.

Once the animal has been slaughtered, the carcass with its labels and/or codes 7 is processed in a cutting plant (it can be in the same physical location or in a different one in which case transportation events can be generated). From here one, the carcass can be transformed in different stages. Once the meat products have been processed, additional traceability events can be generated, such as transportation and distribution, even until the product reaches the end consumer. For this purpose, one or more electronic devices 8 for traceability management can send to the one or more computing devices 3 data from the slaughtered animal, for instance, the animal's identification can be introduced (in this case, labels or codes 7 are used for recognising the animal's identification) and provide additional information such as characteristics of the slaughtering, parts of the animal obtained after cutting, etc.

Any agent or even the end consumer, when he/she received the product can execute an electronic query, through an electronic device 6, to the one or more servers 4 for accessing the traceability events generated, through web interfaces or mobile apps, for instance.

Figure 2 shows diagrammatically a monitoring device 1 in accordance with some embodiments of the invention.

Monitoring device 1 comprises of: one sensor 10 for measuring one or more physical magnitudes of the animals and/or its environment when it is carrying the monitoring device 1; one wireless communication module 11 for data transmission and reception; one or more processors 12; one or more storing units 12; and one or more energy supply sources 12, preferably one or more batteries. Monitoring device 1 is adapted for being placed on an animal as previously described.

Sensor 10 allows for the measurement of one or more physical magnitudes, such as: temperature, humidity, pulse, sound, motion, location, among others. Although motion and position can be determined with a positioning device, in embodiments in which sensor 10 needs to measure one or both, it is preferably done with a sensor 10 of inertial measurement (e.g., accelerometer, gyroscope and/or magnetometer), or event processing the communications of the wireless communication module 11 that allow for triangulating the position. These measurements are stored in one or more storing units 13 and can be processed by the one or more processors 12 for data generation. These measurements, processed or not, are sent to the communication device through the wireless communication module 11.

Figure 3 shows diagrammatically a communication device 2a, 2b in accordance with some embodiments of the invention.

Communication device 2a, 2b comprises of a positioning device 20 set up for determining its location through satellite or wireless communications; a wireless communication module 21 for the data transmission and reception; one or more processors 22; one or more storing units 23; and, optionally, one or more energy supply sources, preferably one or more batteries and/or one or more sensors 25 for measuring physical magnitudes of the animals and/or its environment. En algunos casos como, por ejemplo, cuando el dispositivo de comunicación 2b se instala en una instalacion o vehículo, el dispositivo puede estar alimentado electricamente por medio de una fuente de energía externa al dispositivo, por ejemplo, un motor, la red eléctrica, celulas fotovoltaicas, etc. El dispositivo de comunicación 2a tambien puede estar adaptado para colocarse en un animal tal como se ha explicado anteriormente.

Positioning device 20 can use either electromagnetic waves coming from satellites for determining its location, or triangulation techniques based on wireless communications. The power or arrival time from base stations can be used for the determination of its location.

Communication device 2a, 2b receives packages from the monitoring devices thanks to the wireless communication module 1, stores and processes data through the one or more storage units 23 and one or more processors 22 and sends it with additional data to the one or more computing devices thanks to the same wireless communication module 21. Communication between the communication device 2a, 2b and the monitoring device is achieved through a first communication protocol and the communication between communication device 2^{a}, 2b and the computing device is achieved through a second communication protocol. The first communication protocol is usually used for short-range communication, les tan 500 metres, and the second communication protocol is used for medium or long-range communications, what allows more than 500 metres range, nevertheless, the first communication protocol also allows ranges shorter than 500 metres.

In this text, the word "comprises" and its variants should not be interpreted exclusively, that is to say, they do not exclude the possibility that what has been described includes other elements, steps, etc.

On the other and, this invention is not limited to the particular embodiments that have been described but it also includes, for instance, the variants that could be executed by the average expert on the field (for example, regarding the choice of materials, dimensions, components, settings, etc.), within the scope of the claims.

## Claims

1. A system for generating traceability of an animal (100), comprising: one or more monitoring devices (1), each one adapted for being secured to the animal (100); one or more communication devices (2a,2b); and one or more computing devices (3);
wherein each monitoring device (1) comprises: a first sensor (10) for measuring one or more physical quantities of the animal (100) and/or of an environment in which it is located when the monitoring device (1) is secured to the animal (100); a first wireless communication module (11) for data transmission and reception; a first processor (12); and a first storage unit (13); the first processor (12) being configured for storing measurements of the first sensor (10) and at least one identifier of the animal in the first storage unit (13), and for transmitting at least measurements of the first sensor (10) and the identifier of the animal to the communication device (2a,2b) intermittently and/or after receiving an interrogation from the communication device (2a,2b);
wherein each communication device (2a,2b) comprises: a positioning device (20) configured for determining its position by means of satellites and/or wireless communications; a second wireless communication module (21) for data transmission and reception; a second processor (22); and a second storage unit (23); the second processor (22) being configured for receiving transmissions from the monitoring device (1) when it is in a coverage area of the monitoring device (1), for associating a position determined by the positioning device (20) with the animal (100) of the monitoring device (1) every time transmissions are received, for storing transmissions with an associated position in the second storage unit (13), and for transmitting at least transmissions with an associated position to the one or more computing devices (3) or to a data network to which the one or more computing devices (3) are communicated;
wherein the one or more computing devices (3) comprise: a communication module for data transmission and reception; and one or more processors; the one or more processors being configured for processing transmissions of the monitoring device (1) such that, based on at least the measurements contained in the transmissions and the associated positions, it generates traceability events associated with the animal (100) by means of the identifier of the animal, and for transmitting the traceability events associated with the animal (100) to one or more servers.

2. The system of claim 1, wherein the animal (100) is a first animal; at least one of the one or more communication devices (2a,2b) is adapted for being secured to a second animal (100) and further comprises a second sensor (25) for measuring one or more physical quantities of the second animal (100) and/or of an environment in which it is located when said at least one communication device (2a,2b) is secured to the second animal (100); wherein the second processor (22) of said at least one communication device (2a,2b) is additionally configured for storing measurements of the second sensor (25) and at least one identifier of the second animal in the second storage unit (13), and for transmitting at least the measurements of the second sensor (25) and the identifier of the second animal with an associated position to the one or more computing devices (3) or to a data network to which the one or more computing devices (3) are communicated; wherein the one or more processors are configured for processing the measurements of the second sensor (25) and the associated positions and generating traceability events associated with the second animal (100) by means of the identifier of the second animal, and for transmitting the traceability events associated with the second animal (100) in the one or more servers.

3. The system of any one of the preceding claims, wherein at least one of the one or more communication devices (2a,2b) further comprises one or more second sensors (25) for measuring one or more physical quantities of the animal (100) and/or of an environment in which it is located when the animal (100) actuates or activates the one or more second sensors (25) and/or for measuring one or more physical quantities of an installation (101) or vehicle (102); wherein the second processor (22) of said at least one communication device (2a,2b) is additionally configured for interrogating each of the one or more monitoring devices (1) at least when it detects that it is in a coverage area of each respective monitoring device (1), and is configured for transmitting, together with transmissions with an associated position, measurements of the one or more second sensors (25); and wherein the one or more processors are configured for generating the traceability events also based on the measurements of the one or more second sensors (25).

4. The system of any one of the preceding claims, wherein the one or more servers are configured for providing a digital record book preferably based on blockchain; and wherein the one or more processors are configured for transmitting each traceability event associated with an animal (100) to the one or more servers as a record in the digital record book.

5. The system of any one of the preceding claims, wherein the one or more computing devices (3) are one or more first computing devices (3); wherein the system further comprises one or more second computing devices (3); wherein the one or more second computing devices comprise: a communication module for data transmission and reception; one or more processors; and one or more storage units wherein a predetermined set of thresholds is stored; the one or more processors being configured for downloading the traceability events associated with an animal (100) by means of the identifier of the animal, for processing the downloaded events such that they are compared with the predetermined set of thresholds, for generating a digital certification associated with the animal (100) indicative of the comparison performed, and for transmitting the digital certification associated with the animal to the one or more servers and/or for storing the digital certification associated with the animal (100) in the one or more storage units of the one or more second computing devices.

6. The system of claim 5, wherein the one or more servers is configured for serving digital certifications stored therein upon receiving an electronic request for digital certification consultation, the electronic request including an identification of the animal.

7. A method for generating traceability of an animal (100), comprising:
placing each of one or more monitoring devices (1) on an animal (100) to be traced;
measuring, with a first sensor (10) of each monitoring device (1), one or more physical quantities of the animal (100) and/or of an environment in which it is located when the monitoring device (1) is placed on the respective animal (100);
storing at least the measurements of the first sensor (10) and an identifier of the animal in a first storage unit (13) of the respective monitoring device (1);
transmitting, from each monitoring device (1) to one or more communication devices (2a,2b) wirelessly by means of respective wireless communication modules (11,21) for data transmission and reception, at least the measurement transmissions of the first sensor (10) and the identifier of the respective animal intermittently and/or after receiving an interrogation from the communication device (2a,2b);
determining, at least intermittently, the position of each communication device (2a,2b) by means of a positioning device (20) of the respective communication device (2a,2b), said determination being by means of satellites and/or wireless communications;
storing, in a second storage unit (23) of each communication device (2a,2b), the transmissions received from each monitoring device (1) together with a determined position;
transmitting, from each communication device (2a,2b) to one or more computing devices (3) or to a data network to which the one or more computing devices (3) are communicated, at least the transmissions received together with a determined position;
the one or more computing devices (3) generating traceability events associated with the animal (100), by means of the identifier of the respective animal, based on the transmissions received from the respective monitoring device (1); and
the one or more computing devices (3) transmitting the traceability events associated with each animal (100) to one or more servers.

8. The method of claim 7, wherein each animal (100) to be traced is a first animal (100) to be traced; the method further comprising: placing each of the one or more communication devices (2a,2b) on a second animal (100) to be traced; measuring, with a second sensor (25) of each communication device (2a,2b), one or more physical quantities of the second animal (100) and/or of an environment in which it is located when the communication device (2a,2b) is placed on the respective second animal (100); storing at least the measurements of the second sensor (25) and an identifier of the second animal in the second storage unit (23) of the respective communication device (2a,2b); transmitting, from each communication device (2a,2b) to the one or more computing devices (3) or to the data network to which the one or more computing devices (3) are communicated, at least the measurements of the second sensor (25) together with a determined position and the identifier of the respective second animal; the one or more computing devices (3) generating traceability events associated with the second animal (100), by means of the identifier of the respective second animal, based on the transmissions received from the respective monitoring device (1); and the one or more computing devices (3) transmitting the traceability events associated with each second animal (100) to the one or more servers.

9. The method of claim 8, wherein each first animal (100) is a calf and each second animal (100) is a mother of a calf.

10. The method of any one of the preceding claims, which further comprises: measuring, with one or more second sensors (25) of one or more communication device (2a,2b), one or more physical quantities of the animal (100) and/or of an environment in which it is located with a monitoring device (1) placed when the animal (100) actuates or activates the one or more second sensors (25) and/or one or more physical quantities of an installation (101) or vehicle (102); and interrogating each of the one or more monitoring devices (1) with each of the one or more communication devices (2a,2b) at least when the respective communication device (2a,2b) detects that it is in a coverage area of the respective monitoring device (1); and wherein in the step of transmitting at least the transmissions received together with a determined position, measurements of the one or more second sensors (25) are also transmitted.

11. The method of any one of the preceding claims, which further comprises: transmitting, from an electronic device (6) with a communication module to networks for data transmission and reception, an electronic request for traceability event consultation including an identification of the animal (100); and the electronic device (6) receiving from the one or more servers traceability events associated with the identification of the animal (100) of the electronic request for consultation received.

12. The method of any one of the preceding claims, wherein the one or more computing devices (3) are one or more first computing devices (3); the method further comprising: one or more second computing devices of the one or more servers receiving the traceability events associated with an animal (100) by means of the identifier of the animal; the one or more second computing devices processing the events received such that they are compared with a predetermined set of thresholds; the one or more second computing devices generating a digital certification associated with the animal (100) indicative of the comparison performed; and the one or more second computing devices transmitting the digital certification associated with the animal (100) to the one or more servers and/or storing the digital certification associated with the animal (100) in one or more storage units of the one or more second computing devices.

13. The method of claim 12, wherein in the step of transmitting and/or storing the digital certification, the digital certification is at least transmitted to the one or more servers; and the method further comprising: transmitting, from an electronic device (6) with a communication module to networks for data transmission and reception, an electronic request for digital certification consultation including an identification of the animal; and the electronic device (6) receiving from the one or more servers the digital certificate associated with the identification of the animal of the electronic request for consultation received.

14. The method of any one of the preceding claims, wherein the traceability events comprise one or more of: birth, movement of the animal (100) between farms and premises, weaning of the animal (100), events relating to the health and welfare of the animal (100), entrance of the animal (100) in a feedlot, movement of the animal (100) in the feedlot, exit of the animal (100) from the feedlot, transport of the live animal, slaughter, generation of meat product, and transport of the meat product.

15. The method of any one of the preceding claims, wherein the wireless transmission of each monitoring device (1) to the one or more communication devices (2a,2b) is carried out when a distance between the respective monitoring device (1) and the respective communication device (2a,2b) is less than or equal to 500 meters; and wherein the transmission of each communication device (2a,2b) to the one or more computing devices (3) or to the data network is through a mobile communication network or the Internet.
